(19) 

Europäisches Patentamt
European Patent Office
Office européen des brevets

(11) **EP 4 541 888 A1**

(12) **EUROPEAN PATENT APPLICATION**

(43) Date of publication:
**23.04.2025 Bulletin 2025/17**

(21) Application number: **24191436.5**

(22) Date of filing: **29.07.2024**

(51) International Patent Classification (IPC):
**C12N 9/90** (2006.01)    **C12N 15/52** (2006.01)
**C12N 15/77** (2006.01)    **C12P 19/02** (2006.01)
**C12R 1/15** (2006.01)

(52) Cooperative Patent Classification (CPC):
**C12N 9/90; C12N 15/52; C12N 15/77; C12P 19/02;**
**C12Y 501/03;** C12R 2001/15

(84) Designated Contracting States:
**AL AT BE BG CH CY CZ DE DK EE ES FI FR GB**
**GR HR HU IE IS IT LI LT LU LV MC ME MK MT NL**
**NO PL PT RO RS SE SI SK SM TR**
Designated Extension States:
**BA**
Designated Validation States:
**GE KH MA MD TN**

(30) Priority: **18.10.2023 KR 20230139567**

(71) Applicant: **Daesang Corporation**
**Seoul 03130 (KR)**

(72) Inventors:
• **KIM, Baek Joong**
**07789 Seoul (KR)**

• **CHOI, Eun Seok**
**07789 Seoul (KR)**
• **KIM, Tae Gyun**
**07789 Seoul (KR)**

(74) Representative: **Kehl, Ascherl, Liebhoff & Ettmayr**
**Patentanwälte Partnerschaft mbB**
**Emil-Riedel-Straße 18**
**80538 München (DE)**

Remarks:
The complete document including Reference Table(s) and the Sequence Listing(s) can be downloaded from the EPO website

(54) **ALLULOSE EPIMERASE EXPRESSION CASSETTE AND MANUFACTURING METHOD OF ALLULOSE USING THE SAME**

(57)    An exemplary embodiment of the present disclosure provides an allulose epimerase expression cassette including a polynucleotide encoding allulose epimerase and a promoter which is operably linked thereto and regulates the expression of the allulose epimerase in a *Corynebacterium* genus strain. The promoter consists of a base sequence represented by SEQ ID NO: 1, a base sequence represented by SEQ ID NO: 9, a base sequence represented by SEQ ID NO: 10, or a base sequence represented by SEQ ID NO: 11. According to the exemplary embodiment of the present disclosure, the allulose epimerase expression cassette is hardly operated under a normal environment of culturing a recombinant *Corynebacterium* genus strain and does not induce growth inhibition of the recombinant *Corynebacterium* genus strain. Therefore, the recombinant *Corynebacterium* genus strain transformed with the allulose epimerase expression cassette may grow and proliferate normally until a logarithmic phase and stably and highly express allulose epimerase after the logarithmic phase.

[Figure 1]

EP 4 541 888 A1

**Description**

## CROSS REFERENCE TO RELATED APPLICATIONS

This application is based on and claims priority from Korean Patent Application No. 10-2023-0139567, filed on October 18, 2023, with the Korean Intellectual Property Office, the disclosure of which is incorporated herein in its entirety by reference.

## TECHNICAL FIELD

The present disclosure relates to an allulose epimerase expression cassette and the like, and more particularly, to an allulose epimerase expression cassette capable of highly expressing an allulose enzyme in a stationary phase after a logarithmic phase of a *Corynebacterium* host microorganism without inducing growth inhibition of the *Corynebacterium* genus host microorganism due to a low expression level of the allulose enzyme in an early stage of culture, a recombinant *Corynebacterium* genus strain including the same, a manufacturing method of allulose using the same, and the like.

## BACKGROUND

D-allulose is also called D-psicose as an epimer of carbon at position 3 of fructose. Allulose is a functional monosaccharide that has 70% sweetness compared to sugar (Oshima 2006) but has only 0.3% energy to be applied as a low-calorie sweetener in diet foods (Matsuo et al., 2002). In addition, the allulose has a function of suppressing blood sugar levels by inhibiting glucose absorption and thus may be applied to foods for diabetic patients, foods for nursing patients, and the like, and may suppress the accumulation of abdominal fat due to a function for inhibiting enzyme activity involved in lipid synthesis in the liver and thus may be used for various functional foods such as health foods, and the like (Matsuo et al. 2001; Iida et al. 2008; Hayashi et al. 2010; Hossain et al. 2011). With the characteristics, the allulose is a good source capable of replacing sugar, but belongs to rare sugar, which is monosaccharide that rarely exists in nature, and thus a method of efficiently producing allulose is required to be applied to the food industry.

Representative biological methods for producing allulose include: a method of converting fructose into allulose by directly reacting with D-allulose 3-epimerase, and a method of converting fructose into allulose by reacting with cells of a strain for producing D-allulose 3-epimerase as an intracellular enzyme. A recombinant strain transformed by introduction of a D-allulose 3-epimerase expression cassette is used as a strain for producing the D-allulose 3-epimerase, and *E. coli* is generally used as a host cell. However, the transformed recombinant *E. coli* is not a Generally Recognized As Safe (GRAS) strain and thus is not suitable to be used as a strain for producing food materials. To solve the problem, there is a need for development of a D-allulose 3-epimerase expression cassette suitable for a *Corynebacterium* strain as the GRAS strain.

For example, in Korean Patent Registration No. 10-1656063, there are disclosed a D-allulose 3-epimerase expression cassette suitable for transformation of a *Corynebacterium* genus strain and a method for producing D-allulose using the same. Since the transcription promoter used in the prior art includes a region expressing both a superoxide dismutase (sod) gene and a peptide methionine sulfoxide reductase (MsrA) gene present in a full-length base sequence of *Corynebacterium glutamicum* strain, the use of an expression cassette including the transcription promoter may result in unintended reverse transcription, and further, may adversely affect cell growth due to decreased expression of a target protein or inefficient energy consumption.

## SUMMARY

The present disclosure has been made in an effort to provide an allulose epimerase expression cassette and various uses thereof capable of highly expressing an allulose enzyme in a stationary phase after a logarithmic phase of a *Corynebacterium* genus host microorganism without inducing growth inhibition of the *Corynebacterium* genus host microorganism due to a low expression level of the allulose enzyme in an early stage of culture.

The present inventors manufactured an expression cassette by cloning a promoter region in length of 200 bp for expression of a superoxide dismutase (sod) gene from a genomic DNA of a *Corynebacterium glutamicum* ATCC 13032 strain and sequentially linking the promoter region with allulose epimerase and then manufactured a recombinant allulose epimerase expression vector by inserting the expression cassette into a recombinant shuttle vector. Thereafter, the present inventors manufactured a recombinant *Corynebacterium glutamicum* strain by introducing and transforming the recombinant allulose epimerase expression vector into *Corynebacterium glutamicum* which is a Generally Recognized As Safe (GRAS) strain. The present inventors cultured the recombinant *Corynebacterium glutamicum* strain, and as a result, confirmed that the growth rates during the logarithmic and stationary phases were similar to those of a wild-type *Corynebacterium glutamicum* strain, and D-allulose 3-epimerase was highly expressed by environmental stress from the stationary phase after the logarithmic phase, and then completed the present disclosure.

An exemplary embodiment of the present disclosure provides an allulose epimerase expression cassette including a polynucleotide encoding allulose epimerase and a promoter operably linked thereto. The promoter consists of a base sequence represented by SEQ ID NO: 1, a base sequence represented by SEQ ID NO: 9, a base sequence represented by SEQ ID NO: 10, or a base sequence represented by SEQ ID NO: 11.

Another exemplary embodiment of the present disclosure provides a recombinant expression vector inserted with an allulose epimerase expression cassette.

Yet another exemplary embodiment of the present disclosure provides a recombinant *Corynebacterium* genus strain in which a *Corynebacterium* genus host strain is transformed by introduction of an allulose epimerase expression cassette or a recombinant expression vector inserted with the expression cassette.

Still another exemplary embodiment of the present disclosure provides a manufacturing method of allulose, including adding and reacting a recombinant *Corynebacterium* genus strain to a fructose-containing solution.

According to the exemplary embodiments of the present disclosure, the allulose epimerase expression cassette is hardly operated under a normal environment of culturing a recombinant *Corynebacterium* genus strain and does not induce growth inhibition of the recombinant *Corynebacterium* genus strain. Therefore, the recombinant *Corynebacterium* genus strain transformed with the allulose epimerase expression cassette may grow and proliferate normally until a logarithmic phase and stably and highly express allulose epimerase after the logarithmic phase.

The foregoing summary is illustrative only and is not intended to be in any way limiting. In addition to the illustrative aspects, embodiments, and features described above, further aspects, embodiments, and features will become apparent by reference to the drawings and the following detailed description.

## BRIEF DESCRIPTION OF THE DRAWINGS

FIG. 1 is a schematic diagram illustrating a process of manufacturing a pDS vector, which is a recombinant plasmid vector, based on a pJC 1 vector which is commercial shuttle vector.

FIG. 2 is a schematic diagram illustrating a process of manufacturing a recombinant expression vector pDS_FDPE from a pDS vector which is a recombinant plasmid vector, and FIG. 3 is a cleavage map of the recombinant expression vector pDS_FDPE.

FIG. 4 illustrates a result of comparing growth rates of a wild-type *Corynebacterium glutamicum* strain (indicated as WT) and a recombinant *Corynebacterium glutamicum* strain (indicated as DS00001) in Example 5 of the present disclosure.

FIG. 5 illustrates the D-allulose 3-epimerase activity of a recombinant *Corynebacterium glutamicum* strain by culture time as the conversion rate of fructose to allulose, when inoculating and culturing the recombinant *Corynebacterium glutamicum* strain in a fructose-containing substrate solution in Example 6 of the present disclosure.

## DETAILED DESCRIPTION

In the following detailed description, reference is made to the accompanying drawing, which forms a part hereof. The illustrative embodiments described in the detailed description, drawing, and claims are not meant to be limiting. Other embodiments may be utilized, and other changes may be made, without departing from the spirit or scope of the subject matter presented here.

Hereinafter, the present disclosure will be described in detail.

As used in the present invention, the term 'promoter' refers to a minimum nucleic acid sequence which is operably linked to a target nucleotide sequence that is a transcription target and regulates transcription of the target nucleotide sequence. In addition, the promoter may include promoter components sufficient to express a regulatable promoter-dependent gene induced by a cell-type specific or external signal or agent, and these components may be located at the 5' or 3' portion of the gene. The promoter includes both a conservative promoter and an inducible promoter. The promoter sequence may be derived from prokaryotes, eukaryotes or viruses. In prokaryotes, the promoter is usually defined as a binding site immediately near a transcription start site to which RNA polymerase binds.

As used in the present invention, the term 'homology' refers to the identity to a nucleic acid sequence of a wild type or a mutant having the same activity, and the homology may be compared by calculating the homology between two or more sequences as a percentage (%) visually or by using an easily available comparison program. In addition, the 'homology' is used to represent the identity with an amino acid sequence of a wild type or a mutant having the same activity.

As used in the present invention, the term 'polynucleotide' refers to any non-modified or modified polyribonucleotide (RNA) or polydeoxyribonucleotide (DNA). The polynucleotide includes single- or double-stranded DNA, DNA as a mixture of single- and double-stranded regions, single- and double-stranded RNA, RNA as a mixture of single- and double-stranded regions, or hybrid molecules thereof, but is not limited thereto.

As used in the present invention, the term 'operably linked' is defined as a state in which a promoter sequence and a nucleotide sequence encoding a target protein are functionally linked to each other, so that the promoter may regulate the expression of the target protein. For example, when the promoter is capable of controlling expression of a coding sequence (i.e., when the coding sequence is under the transcriptional regulation of the promoter), the promoter is operably linked to the coding sequence, or when a ribosome binding site is located to promote translation, the ribosome binding site is operably linked to the coding sequence. The coding sequence may be operably linked to a regulatory sequence in either a sense or antisense direction.

As used in the present invention, the term 'recombinant vector' is defined as a recombinant DNA manufactured by cleaving a promoter variant or a target gene using a restriction enzyme and inserting the promoter variant or the target gene into a vector.

As used in the present invention, the term 'expression cassette' refers to a regulatory sequence functionally linked to a nucleotide sequence to be expressed, for example, a polynucleotide sequence encoding allulose epimerase. Accordingly, unlike an expression unit, the expression cassette includes not only a nucleotide sequence that regulates transcription and translation, but also a nucleotide sequence expressed as a protein as a result of transcription and translation.

As used in the present invention, the term 'expression vector' is defined as a DNA sequence necessary for transcription and translation of cloned DNA in a suitable host, and specifically, refers to a genetic construct including essential regulatory elements operably linked to an insert such that the insert is expressed when present within a cell of a subject. The expression vector may be manufactured and purified using standard recombinant DNA technique. The type of expression vector is not particularly limited as long as the expression vector has a function of expressing a desired gene and producing a desired protein in various host cells of prokaryotic and eukaryotic cells. The expression vector includes at least a promoter, a start codon, a gene encoding a desired protein, and a stop codon terminator. In addition, the expression vector may also appropriately include DNA encoding a signal peptide, an additional expression regulatory sequence, untranslated regions at the 5' and 3' sides of a desired gene, a selection marker region, a replicable unit, or the like. The selection marker region may be a selection marker gene of an antibiotic for selecting a desired vector.

As used in the present invention, the term 'recombinant strain' refers to a cell transformed by introducing a polynucleotide encoding at least one target protein or an expression vector having the polynucleotide into a host cell. Methods for preparing a transformant by introducing the expression vector into the host cell include transient transfection, microinjection, transduction, cell fusion, calcium phosphate precipitation, liposome mediated transfection, DEAE dextran-mediated transfection, polybrene-mediated transfection, electroporation, electroinjection, chemical treatment methods such as PEG, methods using gene guns, etc., heat shock, and the like, but are not limited thereto. In addition, the host cell of the recombinant strain is not particularly limited to the type as long as the promoter present in the expression vector may operate properly, and is preferably a prokaryotic organism.

One aspect of the present disclosure relates to an expression cassette capable of inducing high expression of a target protein after a logarithmic phase of a host cell without inducing growth inhibition of the host cell in the early stage of culture.

The allulose epimerase expression cassette according to an exemplary embodiment of the present disclosure includes a polynucleotide encoding allulose epimerase and a promoter operably linked thereto. The promoter is preferably located upstream of the polynucleotide encoding the target protein, allulose epimerase. In addition, the promoter regulates the expression of the allulose epimerase in the *Corynebacterium* genus strain.

The promoter, which is a component of the allulose epimerase expression cassette, is a SOD promoter cloned from a specific portion of the genome of the *Corynebacterium glutamicum* ATCC 13032 strain, and is a polynucleotide corresponding to -200 bp based on the superoxide dismutase (sod) gene. The promoter consists of a base sequence represented by SEQ ID NO: 1. In addition, the promoter includes an analogue of the SOD promoter. The analogue of the SOD promoter may consist of a base sequence represented by SEQ ID NO: 9, a base sequence represented by SEQ ID NO: 10, or a base sequence represented by SEQ ID NO: 11. The specific SOD promoter cloned in the present disclosure has a length of 200 bp, and bases at positions 191 to 200 correspond to a spacer sequence. Since the spacer sequence is a sequence existing between genes and is estimated not to have a significant effect on the expression pattern of the target protein, the space sequence may be partially modified. In the base sequence represented by SEQ ID NO: 9, the base sequence represented by SEQ ID NO: 10, or the base sequence represented by SEQ ID NO: 11, the spacer sequence corresponding to bases at positions 191 to 200 of the base sequence represented by SEQ ID NO: 1 is partially modified. The promoter, which is a component of the allulose epimerase expression cassette according to an exemplary embodiment of the present disclosure, consists of the base sequence represented by SEQ ID NO: 1, the base sequence represented by SEQ ID NO: 9, the base sequence represented by SEQ ID NO: 10, or the base sequence represented by SEQ ID NO: 11, but the equivalent range of the promoter is not necessarily limited thereto. For example, the equivalent range of the SOD promoter of the present disclosure includes a sequence having the substantial identity to the base sequence represented by SEQ ID NO: 1, the base sequence represented by SEQ ID NO: 9, the base sequence represented by SEQ ID NO: 10, or the base sequence represented by SEQ ID NO: 11. The substantial identity means that any other sequences have a sequence homology of 70% or more, 90% or more, or 98% or more the base sequence represented by SEQ ID NO: 1, the base sequence represented by SEQ ID NO: 9, the base sequence represented by SEQ ID NO: 10, or the base sequence represented by SEQ ID NO: 11 by aligning other sequences to maximally correspond to the base sequence represented by SEQ ID NO: 1, the base sequence represented by SEQ ID NO: 9, the base sequence represented by SEQ ID NO: 10, or the base sequence represented by SEQ ID NO: 11 and analyzing the sequences thereof. It will be easily appreciated that those skilled in the art may manufacture a polynucleotide having the same or similar activity within the range having the substantial homology by substituting, adding or deleting one or more bases in the base sequence of the SOD promoter using a genetic recombination technique and the like known in the art. Comparison of such a homology may be performed by calculating a homology between two or more sequences as a percentage (%) using a commercially available computer program.

The polynucleotide encoding allulose epimerase, which is a component of the allulose epimerase expression cassette, is not particularly limited to the type as long as the polynucleotide is a polynucleotide encoding an enzyme having the activity of converting fructose into allulose. For example, the allulose epimerase may be derived from *Flavonifractor plautii, Clostridium scindens, Treponema primitia, Ensifer adhaerens* or *Ruminococcus torques,* and preferably derived from *Flavonifractor plautii* when considering the conversion activity of fructose into allulose. Specifically, the allulose epimerase may consist of an amino acid sequence represented by SEQ ID NO: 3, an amino acid sequence represented by SEQ ID NO: 13, or an amino acid sequence represented by SEQ ID NO: 15. The allulose epimerase consisting of the amino acid sequence represented by SEQ ID NO: 3 is a wild-type enzyme derived from *Flavonifractor plautii.* In the allulose epimerase consisting of the amino acid sequence represented by SEQ ID NO: 13, tryptophan (Trp) at position 29 in the amino acid sequence represented by SEQ ID NO: 3 is substituted with lysine (Lys), glycine (Gly) at position 216 is substituted with serine (Ser), and simultaneously, methionine (Met) at position 234 is substituted with isoleucine (Ile). In the allulose epimerase consisting of the amino acid sequence represented by SEQ ID NO: 15, tryptophan (Trp) at position 29 in the amino acid sequence represented by SEQ ID NO: 3 is substituted with lysine (Lys), alanine (Ala) at position 77 is substituted with serine (Ser), glycine (Gly) at position 216 is substituted with serine (Ser), and simultaneously, methionine (Met) at position 234 is substituted with isoleucine (Ile). The polynucleotide encoding the allulose epimerase is not particularly limited to the type, and preferably, may consist of a base sequence represented by SEQ ID NO: 2, or include a base sequence having 70% or more, 80% or more, 90% or more, 95% or more, or 99% or more of a homology with the base sequence represented by SEQ ID NO: 2. The base sequence represented by SEQ ID NO: 2 is a polynucleotide encoding allulose epimerase consisting of the amino acid sequence represented by SEQ ID NO: 3. In addition, the polynucleotide encoding the allulose epimerase may consist of a base sequence represented by SEQ ID NO: 12 or a base sequence represented by SEQ ID NO: 14. The base sequence represented by SEQ ID NO: 12 is a polynucleotide encoding allulose epimerase consisting of the amino acid sequence represented by SEQ ID NO: 13, and the base sequence represented by SEQ ID NO: 14 is a polynucleotide encoding allulose epimerase consisting of the amino acid sequence represented by SEQ ID NO: 15. With respect to the allulose epimerase and the polynucleotide encoding the same, the present disclosure includes the contents disclosed in Korean Patent Registration Nos. 10-1919713, 10-2187354, 10-1656063, 10-1695830, 10-2189458, 10-1539097, 10-1539096, 10-1455759, 10-1318422, etc.

The allulose epimerase expression cassette according to an exemplary embodiment of the present disclosure may further include at least one sequence selected from the group consisting of a replication origin, a multi cloning site (MCS) for cloning a target protein gene, a transcription termination sequence, and a selection marker. The selection marker is used to select cells transformed with a vector, and may be used with markers that impart selectable phenotypes such as drug resistance, auxotrophy, resistance to cytotoxic agents, or expression of surface proteins. For example, the selection marker may be an antibiotic resistance gene marker, such as a Kanamycin antibiotic resistance gene or an Ampicillin antibiotic resistance gene.

One aspect of the present disclosure relates to various uses of an allulose epimerase expression cassette. The allulose epimerase expression cassette may be used for recombinant vectors, recombinant strains, manufacture of allulose from fructose, etc.

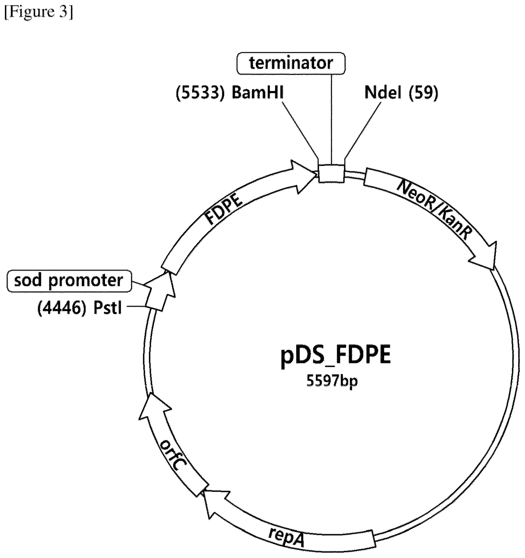

The recombinant vector according to an exemplary embodiment of the present disclosure is a recombinant expression vector inserted with the above-described allulose epimerase expression cassette. The recombinant expression vector preferably has a cleavage map of FIG. 3. The recombinant expression vector having the cleavage map of FIG. 3 has a structure in which an origin of replication, a promoter SOD consisting of a base sequence represented by SEQ ID NO: 1, a polynucleotide FDPE encoding allulose epimerase consisting of a base sequence represented by SEQ ID NO: 2, a transcription terminator, and a Kanamycin resistance gene marker NeoR/KanR consisting of a base sequence represented by SEQ ID NO: 4 are sequentially linked to each other.

The recombinant strain according to an exemplary embodiment of the present disclosure is a recombinant *Corynebacterium* genus strain in which a host cell is transformed by introduction of the allulose epimerase expression cassette described above or the recombinant expression vector inserted with the expression cassette. The host strain used to manufacture the recombinant *Corynebacterium* genus strain is not particularly limited to the type as long as the host strain is a *Corynebacterium* genus strain, and for example, may be selected from the group consisting of *Corynebacterium glutamicum, Corynebacterium acetoglutamicum, Corynebacterium acetoacidophilum, Corynebacterium thermoaminogenes, Corynebacterium melassecola,* and *Corynebacterium efficiens.*

A manufacturing method of allulose from fructose according to an exemplary embodiment of the present disclosure includes adding and reacting a recombinant *Corynebacterium* genus strain to a fructose-containing solution. The fructose-containing solution may further include metal ions such as $Ca^{2+}$ and $Mn^{2+}$ to promote the activity of allulose epimerase. In addition, in the manufacturing method of allulose from fructose, the reaction temperature is in the range of 50 to 70°C, preferably 55 to 65°C, and more preferably 60 to 65°C when considering smooth enzyme expression of the recombinant strain, and the stability and maximum activity of the enzyme, and the reaction pH is in the range of 6.5 to 8, preferably 6.5 to 7.5, and more preferably 6.5 to 7. In addition, in the manufacturing method of allulose from fructose, the concentration of fructose of the fructose-containing solution is not particularly limited, but preferably 5 to 75% (w/w), and more preferably 10 to 55% (w/w) based on the total weight of the fructose-containing solution when considering the productivity and economy.

Hereinafter, the present disclosure will be described in more detail with reference to Examples. However, the following Examples are only for clearly illustrating the technical features of the present disclosure, but do not limit the protection scope of the present disclosure.

**Example 1: Amplification and obtainment of promoter sequences for overexpression of D-allulose 3-epimerase**

In order to obtain a polynucleotide fragment of a promoter region for expression of a superoxide dismutase (sod) gene from a *Corynebacterium glutamicum* ATCC 13032 strain, a genomic DNA was extracted from the *Corynebacterium glutamicum* ATCC 13032 strain. Thereafter, PCR was performed using the extracted genomic DNA as a template and a primer set described in Table 1 below, and the amplified promoter fragment was obtained. The amplified promoter fragment was cloned into a cloning vector and the base sequence was analyzed, and as a result, it was confirmed that the promoter fragment was a polynucleotide fragment having a length of 200 bp and consisting of a base sequence represented by SEQ ID NO: 1.

[Table 1]

| Amplification target | Primer type (SEQ ID NO) | Primer base sequence (5'→3') | Restriction enzyme site |
|---|---|---|---|
| SOD promoter | Forward (SEQ ID NO: 5) | agtcactgcagaccctacttagctgccaa | pstI |
| | Reverse (SEQ ID NO: 6) | aaacctacgaaaggatttttacccatgtgtcagatg aacccgattggaa | |

In general, an exact location relationship of an SOD promoter region based on a superoxide dismutase (sod) gene was not defined, but a region obtained using the primer set in Table 1 above was confirmed to be -200 bp from the superoxide dismutase (sod) gene. On the other hand, an expression regulatory sequence described in Korean Patent Registration No. 10-1656063 was -300 bp based on the superoxide dismutase (sod) gene.

**Example 2: Amplification and obtainment of polynucleotide sequences encoding D-allulose 3-epimerase**

A genomic DNA was extracted from a *Flavonifractor plautii* KCTC 5970 strain to clone a gene encoding D-allulose 3-epimerase. Thereafter, PCR was performed using the extracted genomic DNA as a template and a primer set described in Table 2 below, and the amplified D-allulose 3-epimerase gene fragment was obtained. The amplified gene fragment was cloned into a cloning vector and the base sequence was analyzed, and as a result, it was confirmed that the gene fragment was a polynucleotide fragment having a length of 885 bp and consisting of a base sequence represented by SEQ ID NO: 2, which was named a FDPE gene. The D-allulose 3-epimerase decoded by the polynucleotide consisting of the base sequence represented by SEQ ID NO: 2 was a wild-type enzyme and consisted of an amino acid sequence represented by SEQ ID NO: 3.

[Table 2]

| Amplification target | Primer type (SEQ ID NO) | Primer base sequence (5'→3') | Restriction enzyme site |
|---|---|---|---|
| D-allulose 3-epimerase gene FDPE | Forward (SEQ ID NO: 7) | ttccaatcgggttcatctgacacatgggtaaaaaatcct ttcgtaggttt | |
| | Reverse (SEQ ID NO: 8) | aattcggatccttacgcggtcagctccttg | BamHI |

**Example 3: Manufacture of expression vector inserted with SOD promoter fragment and D-allulose 3-epimerase gene**

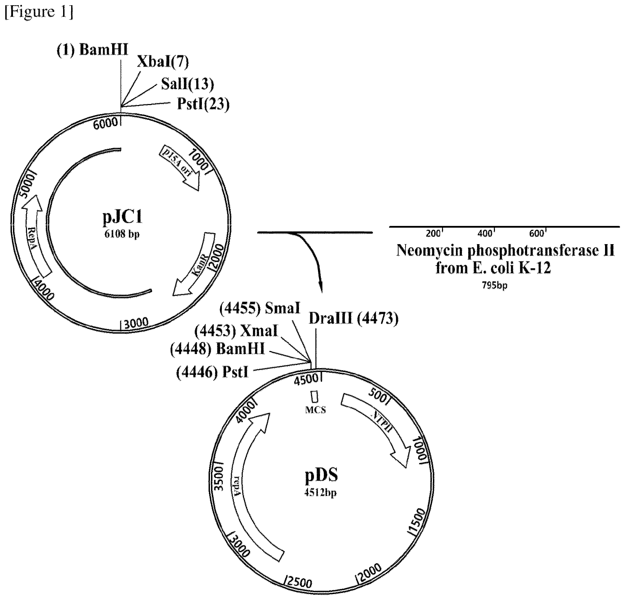

An antibiotic resistance gene, neomycin phospho-transferase I (NTPI) of a pJC1 vector which was a commercial shuttle vector was replaced with an NTPII gene derived from *E. coli* K-12, and a multi cloning site (MCS) and the like were inserted to manufacture a pDS vector, which was a recombinant plasmid vector. FIG. 1 is a schematic diagram illustrating a process of manufacturing a pDS vector, which is a recombinant plasmid vector, based on a pJC1 vector which is commercial shuttle vector. The pDS vector included a replication initiation protein (repA) gene, an antibiotic (Kanamycin) resistance gene derived from *E. coli* K-12 consisting of A base sequence represented by SEQ ID NO: 4, etc.

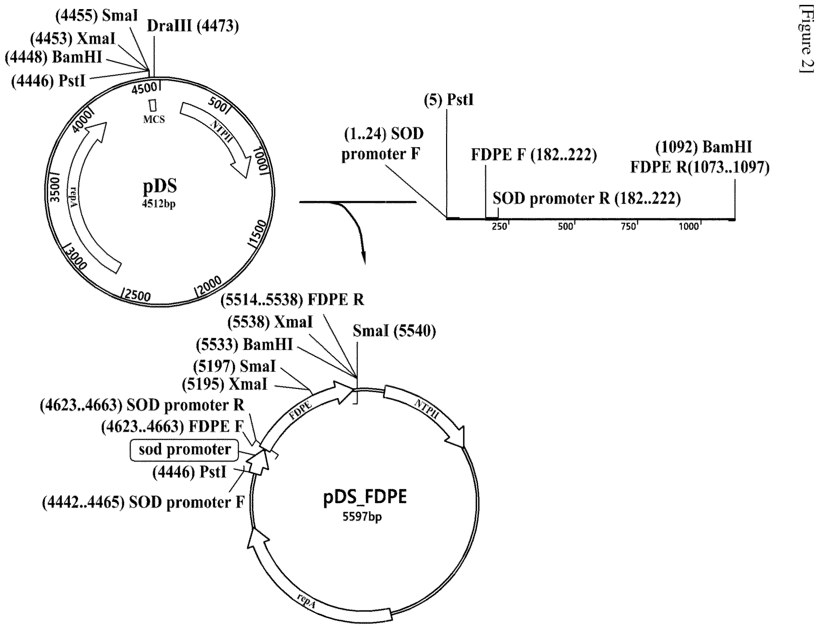

Thereafter, the 200 bp-long SOD promoter DNA fragment and the FDPE gene fragment obtained above were mixed and PCR was performed for 10 cycles. Thereafter, a SOD promoter forward primer described in Table 1 and a FDPE reverse primer described in Table 2 were added and PCR was performed for 15 cycles to obtain a fusion DNA fragment combined with the SOD promoter and the FDPE gene. Thereafter, the ends of the fusion DNA fragment were cleaved using restriction enzymes PstI and BamHI, and the fusion DNA fragment was inserted into PstI and BamHI restriction enzyme sites of the multi cloning site (MCS) of the pDS vector to manufacture a recombinant expression vector pDS_FDPE. FIG. 2 is a schematic diagram illustrating a process of manufacturing a recombinant expression vector pDS_FDPE, from a pDS vector which is a recombinant plasmid vector, and FIG. 3 is a cleavage map of the recombinant expression vector pDS_FDPE. As shown in FIG. 3, the recombinant expression vector pDS_FDPE included a replication initiation protein (repA) gene, a replication origin, a multiple cloning site (MCS) such as a restriction enzyme PstI site, a BamHI site, and an NdeI site, an SOD promoter, a target gene FDPE, a transcription terminator, and an antibiotic resistance gene marker.

**Example 4: Manufacture of recombinant strain transformed by introduction of recombinant expression vector pDS_FDPE and confirmation of activity of D-allulose 3-epimerase**

The recombinant expression vector pDS_FDPE manufactured above was introduced into a *Corynebacterium glutamicum* competent cell using electroporation, and antibiotic resistance was confirmed to select a transformed recombinant *Corynebacterium glutamicum* strain. The selected recombinant *Corynebacterium glutamicum* strain was inoculated into a 2YT medium (containing 16 g/L Trytone, 10 g/L Yeast Extract, and 5 g/L NaCl) and cultured at 30°C for 40 hr. The culture medium was removed by centrifugation, suspended in a 0.85% (w/w) NaCl solution, and centrifuged again to obtain cells of the recombinant *Corynebacterium glutamicum* strain. A cell suspension was prepared by adding the obtained cells of the recombinant *Corynebacterium glutamicum* strain to a 50 mM Tris-HCl buffer (pH 7.0) with 1 mM $MnSO_4$ concentration at an amount of 0.06% (w/w). Thereafter, a 50 mM Tris-HCl buffer (pH 7.0) with 20% (w/w) fructose concentration and 1 mM $MnSO_4$ concentration was mixed with the cell suspension of the recombinant *Corynebacterium glutamicum* strain in a volume ratio of 1 : 1, and an enzyme conversion reaction was performed at 65°C for 1 hr, a 1 M hydrochloric acid solution was added to lower pH to 2 or lower and then the reaction was terminated. Thereafter, a reaction product solution was centrifuged to remove the cells and foreign substances and recover a supernatant and the supernatant was filtered through a 0.45 μm syringe filter to prepare a sample. Thereafter, the activity of the D-allulose 3-epimerase of the transformed recombinant *Corynebacterium glutamicum* strain was confirmed by measuring the concentrations of allulose and fructose in the sample using high-performance liquid chromatography (HPLC). High-performance liquid chromatography (HPLC) analysis conditions were as follows.

* Column: 87C (BIO-RAD)
* Column temperature: 80°C
* Mobile phase: ion exchange water
* Flow rate: 0.6 ml/min
* Detector: Refractive Index Detector (Agilent 1260 TID)

**Example 5: Comparison of growth rates of wild-type *Corynebacterium glutamicum* strain and recombinant *Corynebacterium glutamicum* strain**

A wild-type *Corynebacterium glutamicum* strain and a recombinant *Corynebacterium glutamicum* strain were inoculated in a 2YTG medium (containing 16 g/L Trytone, 10 g/L Yeast Extract, 5 g/L NaCl, 20 g/L dextrose; pH 7), respectively, and seed-cultured at 30°C for 16 hr. Thereafter, the seed-cultured solution containing the cells was inoculated into a 2YTG medium at a 1% (v/v) amount and sampled for each time during a main culture at 30°C. The growth rates of the strains were evaluated by measuring an optical density at 600 nm of the sampled sample.

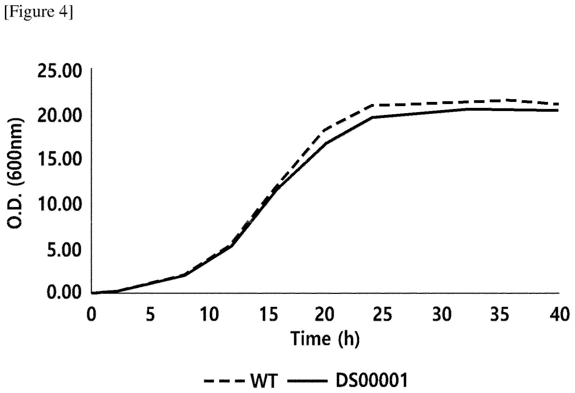

FIG. 4 is a result of comparing growth rates of a wild-type *Corynebacterium glutamicum* strain (indicated as WT) and a recombinant *Corynebacterium glutamicum* strain (indicated as DS00001) in Example 5 of the present disclosure. As illustrated in FIG. 4, the growth rates of the wild-type *Corynebacterium glutamicum* strain and the recombinant *Corynebacterium glutamicum* strain did not show a significant difference. The recombinant *Corynebacterium glutamicum* strain manufactured in the present disclosure did not exhibit overexpression of D-allulose 3-epimerase by the SOD promoter in the early stage of culture and exhibited logarithmic phase and stationary phase at levels similar to those of the wild-type *Corynebacterium glutamicum* strain.

**Example 6: Activity of D-allulose 3-epimerase of recombinant *Corynebacterium glutamicum* strain by culture time**

A recombinant *Corynebacterium glutamicum* strain was inoculated into a 2YT medium (containing 16 g/L Trytone, 10 g/L Yeast Extract, and 5 g/L NaCl) and cultured at 30°C for 40 hr. The culture medium was removed by centrifugation, suspended in a 0.85% (w/w) NaCl solution, and centrifuged again to obtain cells of the recombinant *Corynebacterium glutamicum* strain. A cell suspension was prepared by adding the obtained cells of the recombinant *Corynebacterium glutamicum* strain to 50 mM Tris-HCl buffer (pH 7.0) having a 1 mM $MnSO_4$ concentration at an amount of 0.06% (w/w). Thereafter, a 50 mM Tris-HCl buffer (pH 7.0) with 20% (w/w) fructose concentration and 1 mM $MnSO_4$ concentration was mixed with the cell suspension of the recombinant *Corynebacterium glutamicum* strain in a volume ratio of 1 : 1, and while an enzyme conversion reaction was performed at 65°C, the activity of D-allulose 3-epimerase of the recombinant *Corynebacterium glutamicum* strain was measured according to a reaction time. When a predetermined reaction time was reached, the reaction product solution was added with a 1 M hydrochloric acid solution to lower the pH to 2 or lower and the reaction was terminated. Thereafter, the reaction product solution was centrifuged to remove the cells and foreign substances and recover a supernatant, and the supernatant was filtered through a 0.45 μm syringe filter to prepare a sample. Thereafter, the concentrations of allulose and fructose in the sample were measured using high-performance liquid chromatography (HPLC), and the conversion rate of fructose to allulose was calculated.

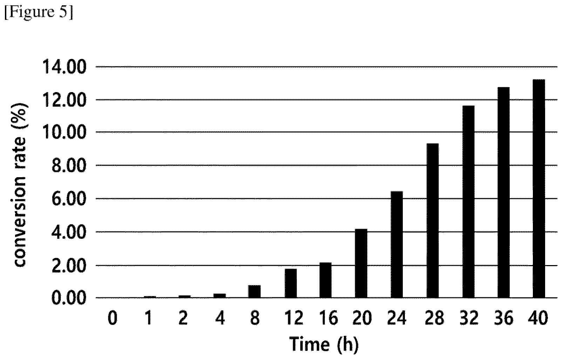

FIG. 5 illustrates the D-allulose 3-epimerase activity of a recombinant *Corynebacterium glutamicum* strain by culture time as the conversion rate of fructose to allulose, when inoculating and culturing the recombinant *Corynebacterium glutamicum* strain in a fructose-containing substrate solution in Example 6 of the present disclosure. As illustrated in FIG. 5, the recombinant *Corynebacterium glutamicum* strain showed no growth inhibition due to overexpression of D-allulose 3-epimerase in the early stage of culture, and was shown to highly express D-allulose 3-epimerase from the stationary phase after sufficient growth to the logarithmic phase.

**Example 7: Production of allulose using recombinant *Corynebacterium glutamicum* strain in commercial substrate-containing solution**

500 g (equivalent to about 380 mL) of a high-fructose corn syrup (HFCS; sugar composition: 55 wt% of fructose, 45 wt% of glucose) having a solid concentration of about 75 Brix, 500 g of a cell suspension of a recombinant *Corynebacterium glutamicum* strain, and 0.2 g of $MnSO_4 \cdot 4H_2O$ were mixed, the pH was adjusted to about 7 using a 4% NaOH aqueous solution, and then the enzyme conversion reaction was performed at 65°C under a stirring condition of 200 rpm. While the enzymatic conversion reaction was performed, the pH of the reaction system was maintained at 7 using a 4% NaOH aqueous solution. The sampling was performed at each enzyme conversion reaction time, and a 10% HCl aqueous solution was added to the sampled sample to stop the enzyme conversion reaction. Thereafter, the sampled sample was diluted approximately 25 times using distilled water, filtered through a 0.45 μm syringe filter, and then the filtrate was analyzed using high-performance liquid chromatography (HPLC) to measure the conversion rate. The conversion rate was calculated using the following formula.

$$\text{Conversion rate (\%)} = \frac{\text{concentration of produced allulose}}{\text{concentration of remaining fructose} + \text{concentration of produced allulose}} \times 100$$

## EP 4 541 888 A1

The results of measuring the conversion rate of fructose into allulose for each reaction time were summarized in Table 3 below, when the recombinant *Corynebacterium glutamicum* strain of the present disclosure was inoculated into high-fructose syrup, a commercial substrate, and an enzymatic conversion reaction was performed.

[Table 3]

| Reaction elapse time (hr) | Conversion rate (%) of fructose into allulose |
| --- | --- |
| 0 | 0 |
| 1 | 13.21 |
| 2 | 21.66 |
| 4 | 26.53 |
| 6 | 28.82 |
| 8 | 29.94 |
| 10 | 30.01 |
| 12 | 29.89 |
| 14 | 29.93 |

As shown in Table 3 above, when allulose was manufactured from the high-fructose syrup using the recombinant *Corynebacterium glutamicum* strain of the present disclosure, the conversion rate of 30% was reached within about 12 hr.

As described above, the present disclosure has been described through examples above, but the present disclosure is not necessarily limited thereto, and various modifications may be made without departing from the scope and spirit of the present disclosure. Therefore, the scope of the present disclosure is not limited to a specific embodiment disclosed as the best form, and it should be construed as including all embodiments falling within appended claims of the present disclosure.

## Claims

1.    An allulose epimerase expression cassette comprising a polynucleotide encoding allulose epimerase and a promoter operably linked thereto,
wherein the promoter consists of a base sequence represented by SEQ ID NO: 1, a base sequence represented by SEQ ID NO: 9, a base sequence represented by SEQ ID NO: 10, or a base sequence represented by SEQ ID NO: 11.

2.    The allulose epimerase expression cassette of claim 1, wherein the allulose epimerase is derived from *Flavonifractor plautii, Clostridium scindens, Treponema primitia, Ensifer adhaerens* or *Ruminococcus torques.*

3.    The allulose epimerase expression cassette of claim 1, wherein the allulose epimerase consists of an amino acid sequence represented by SEQ ID NO: 3.

4.    The allulose epimerase expression cassette of claim 1, wherein the polynucleotide encoding the allulose epimerase consists of a base sequence represented by SEQ ID NO: 2.

5.    The allulose epimerase expression cassette of claim 1, further comprising:
at least one sequence selected from the group consisting of a replication origin, a multi cloning site (MCS) for cloning a target protein gene, a transcription termination sequence, and a selection marker.

6.    A recombinant expression vector inserted with the expression cassette of any one of claims 1 to 5.

7.    The recombinant expression vector of claim 6, wherein the recombinant expression vector has a cleavage map of FIG. 3.

8.    A recombinant *Corynebacterium* genus strain in which a *Corynebacterium* genus host strain is transformed by introduction of the expression cassette of any one of claims 1 to 5 or a recombinant expression vector inserted with the expression cassette.

9. The recombinant *Corynebacterium* genus strain of claim 8, wherein the *Corynebacterium* genus host strain is selected from the group consisting of *Corynebacterium glutamicum, Corynebacterium acetoglutamicum, Corynebacterium acetoacidophilum, Corynebacterium thermoaminogenes, Corynebacterium melassecola,* and *Corynebacterium efficiens.*

10. A manufacturing method of allulose from fructose, comprising adding and reacting the recombinant *Corynebacterium* genus strain of claim 9 to a fructose-containing solution.

[Figure 1]

[Figure 2]

[Figure 3]

[Figure 4]

[Figure 5]

Europäisches
Patentamt

European
Patent Office

Office européen
des brevets

## EUROPEAN SEARCH REPORT

Application Number

EP 24 19 1436

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | EP 3 214 176 A2 (SAMYANG CORP [KR]) 6 September 2017 (2017-09-06) * abstract * * paragraph [0087] * * claims 1,17,18,21,22,29,32,36 * * sequences 1,14 * | 1-10 | INV. C12N9/90 C12N15/52 C12N15/77 C12P19/02 ADD. C12R1/15 |
| A | WO 2023/090495 A1 (DAESANG CORP [KR]) 25 May 2023 (2023-05-25) * paragraphs [0019], [0041] * * sequences 1,4 * | 1-10 | |
| A | US 2020/270587 A1 (LEE SANG YUP [KR] ET AL) 27 August 2020 (2020-08-27) * abstract * * example 9 * * sequence 64 * | 1-10 | |
| X,P | KR 2024 0104295 A (DAESANG CORP [KR]) 5 July 2024 (2024-07-05) * the whole document * | 1-10 | |

TECHNICAL FIELDS
SEARCHED (IPC)

C12N
C40B
C12P
C12R

The present search report has been drawn up for all claims

| Place of search | Date of completion of the search | Examiner |
|---|---|---|
| The Hague | 14 January 2025 | Sonnerat, Isabelle |

CATEGORY OF CITED DOCUMENTS

X : particularly relevant if taken alone
Y : particularly relevant if combined with another
   document of the same category
A : technological background
O : non-written disclosure
P : intermediate document

T : theory or principle underlying the invention
E : earlier patent document, but published on, or
   after the filing date
D : document cited in the application
L : document cited for other reasons

........................................................................

& : member of the same patent family, corresponding
   document

EPO FORM 1503 03.82 (P04C01)

## ANNEX TO THE EUROPEAN SEARCH REPORT
## ON EUROPEAN PATENT APPLICATION NO.

EP 24 19 1436

14-01-2025

| Patent document cited in search report | | Publication date | Patent family member(s) | | | Publication date |
|---|---|---|---|---|---|---|
| EP 3214176 | A2 | 06-09-2017 | CN | 107257856 | A | 17-10-2017 |
| | | | EP | 3214176 | A2 | 06-09-2017 |
| | | | ES | 2796749 | T3 | 30-11-2020 |
| | | | JP | 6563013 | B2 | 21-08-2019 |
| | | | JP | 2017531437 | A | 26-10-2017 |
| | | | US | 2016138005 | A1 | 19-05-2016 |
| | | | US | 2019169591 | A1 | 06-06-2019 |
| | | | US | 2019169592 | A1 | 06-06-2019 |
| | | | WO | 2016068656 | A2 | 06-05-2016 |
| WO 2023090495 | A1 | 25-05-2023 | CN | 116917481 | A | 20-10-2023 |
| | | | EP | 4249595 | A1 | 27-09-2023 |
| | | | JP | 7535192 | B2 | 15-08-2024 |
| | | | JP | 2023554112 | A | 26-12-2023 |
| | | | KR | 20230073739 | A | 26-05-2023 |
| | | | US | 2024052389 | A1 | 15-02-2024 |
| | | | WO | 2023090495 | A1 | 25-05-2023 |
| US 2020270587 | A1 | 27-08-2020 | CN | 111607544 | A | 01-09-2020 |
| | | | KR | 20200104028 | A | 03-09-2020 |
| | | | US | 2020270587 | A1 | 27-08-2020 |
| KR 20240104295 | A | 05-07-2024 | NONE | | | |

EPO FORM P0459

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- KR 1020230139567
- KR 101656063
- KR 101919713
- KR 102187354
- KR 101695830
- KR 102189458
- KR 101539097
- KR 101539096
- KR 101455759
- KR 101318422